# FASCICULE DE BREVET EUROPEEN

(11) **EP 2 206 531 B1**
(45) Date de publication et mention de la délivrance du brevet: **09.03.2011**
(21) Numéro de dépôt: 09176955.4
(22) Date de dépôt: 24.11.2009
(51) Int. Cl.: A61N 1/362, A61N 1/368, A61N 1/365

(54) **Dispositif médical actif du type prothèse cardiaque implantable pour le traitement de l'insuffisance cardiaque par ajustement contrôlé des délais atrioventriculaire et interventriculaire**
Aktive medizinische Vorrichtung vom Typ implantierbare Herzprothese für die Behandlung von Herzinsuffizienz durch kontrollierte Anpassung der atrioventrikulären und intraventrikulären Durchblutungszeiten
Active medical device for treating cardiac heart failure by controlled adjustment of atrioventricular and interventricular delays

(30) Priorité: 09.01.2009 FR 0900060
(43) Date de publication de la demande: 14.07.2010
(73) Titulaire: ELA MEDICAL, 92541 Montrouge (FR)
(72) Inventeur: Limousin, Marcel, 75014 Paris (FR)
(74) Mandataire: Dupuis-Latour, Dominique

(56) Documents cités:
- WO-A-95/19806
- WO-A-99/30777
- WO-A-2005/011475
- US-A- 5 156 154
- US-A1- 2003 208 240
- US-A1- 2004 220 636
- US-A1- 2008 243 202

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement les implants destinés à traiter l'insuffisance cardiaque, en alternative ou en complément au traitement des troubles du rythme cardiaque.

En effet, il a été proposé de traiter par stimulation des cavités cardiaques certains troubles de contraction myocardique observés chez des patients en insuffisance cardiaque, ces troubles pouvant être spontanés ou induits par une stimulation traditionnelle. Cette thérapie a permis d'observer des résultats souvent spectaculaires pour des patients en insuffisance cardiaque non améliorée par les traitements classiques.

Cette thérapie vise à resynchroniser la contraction des cavités cardiaques (oreillette et ventricule, et les deux ventricules entre eux) de manière à améliorer l'état du patient par optimisation des différentes phases du cycle hémodynamique, ce cycle comprenant : pré-éjection, contraction isovolumique, éjection systolique, relaxation isovolumique, et enfin remplissage de la cavité.

Pour optimiser l'hémodynamique cardiaque, il convient :
- d'assurer un temps de remplissage maximal entre l'instant de la fermeture de la valve aortique et celui de la fermeture de la valve mitrale, et
- de prévoir un délai systolique assurant un temps d'éjection maximal par rapport au temps de pré-éjection, condition d'efficacité de la phase systolique.

Cette optimisation peut être réalisée par un ajustement du délai atrioventriculaire et/ou du délai interventriculaire.

Le délai atrioventriculaire, ci-après "délai AV" (DAV ou AVD), est le délai séparant, au cours d'un même cycle cardiaque, un événement atrial (contraction de l'oreillette, spontanée ou stimulée par le dispositif) de la stimulation consécutive du ventricule.

Le délai interventriculaire, ci-après "délai VV" (DVV ou VVD), est le délai séparant, au cours d'un même cycle cardiaque, deux stimulations ventriculaires appliquées conjointement à chacun des deux ventricules droit et gauche, ce délai étant ajusté de manière à resynchroniser la contraction des ventricules - technique dite CRT (*Cardiac Resynchronisation Therapy*) ou BVP (*Bi-Ventricular Pacing*).

Le EP 0 862 927 A1 (ELA Medical) décrit une méthode pour ajuster ces paramètres DAV et DVV en fonction de la contraction ventriculaire, détectée par un capteur permettant de mesurer des variations représentatives soit du volume, soit du mouvement des fibres musculaires ventriculaires en début de systole et/ou de détecter l'ouverture d'une ou des deux valvules sigmoïdes, de manière à en déterminer l'instant d'ouverture. Cette détection peut être opérée par divers types de capteurs connus tels que capteurs de mesure de l'impédance électrique du myocarde, capteurs de mesure de la contractilité, capteurs de mesure du volume ventriculaire par magnétométrie, capteurs de détection d'ouverture de la valvule par transducteur à ultrasons, etc., ces capteurs pouvant être montés sur une sonde de stimulation endocavitaire.

L'expérience clinique démontre toutefois que l'optimisation d'un paramètre hémodynamique, par exemple le temps de remplissage, ou le temps de pré-éjection gauche, n'est pas suffisant.

En effet, il existe une interrelation étroite entre les différentes phases du cycle cardiaque, et les méthodes jusqu'à présent utilisées ne permettent pas de corréler l'évolution de toutes les phases cardiaques, ni de trouver le meilleur réglage des paramètres DAV et/ou DVV.

Le point de départ de l'invention est la constatation du fait que, pour obtenir une amélioration de l'état hémodynamique d'ensemble du patient, il ne suffit pas d'optimiser l'une des phases cardiaques, mais il faut rechercher le meilleur compromis pour l'ensemble du cycle, sans que l'optimisation d'une partie du cycle cardiaque se fasse au détriment des autres parties du cycle. Ainsi :
- l'optimisation de la systole ne doit pas réduire le temps de remplissage ; et
- l'optimisation du temps de remplissage ne doit pas réduire l'efficacité de la systole.

L'un des buts de la présente invention est d'utiliser une combinaison de plusieurs paramètres hémodynamiques, en gérant leur interrelation pour optimiser l'ajustement des paramètres de stimulation.

Le dispositif de l'invention est du type connu comportant : des moyens de stimulation atrioventriculaire et/ou biventriculaire, aptes à appliquer au cours d'un même cycle cardiaque un délai atrioventriculaire AV entre un événement atrial et une stimulation ventriculaire, et/ou un délai interventriculaire VV entre deux stimulations ventriculaires ; des moyens capteurs aptes à délivrer au cours d'un même cycle cardiaque au moins deux paramètres hémodynamiques différents corrélés à des intervalles de temps représentatifs de la succession des phases systoliques et des phases diastoliques ; des moyens pour ajuster le délai AV et/ou le délai VV en fonction dudit paramètre hémodynamique ; et des moyens, opérant dynamiquement et de façon répétée sur plusieurs cycles cardiaques, pour déterminer lesdits au moins deux paramètres hémodynamiques différents, vérifier la concordance de ces paramètres hémodynamiques avec au moins un critère prédéterminé, et dans la négative, réajuster en réponse le délai AV et/ou le délai VV.

Des dispositifs de ce type sont connus de l'art, comme le montre les documents US-A-5 156 154 et US-A-2008/0243202 qui divulguent l'utilisation du temps d'éjection ventriculaire gauche, de la période de pré-éjection et du rapport entre ces valeurs pour ajuster différents intervalles de stimulation.

De façon caractéristique de l'invention, ledit au moins un critère prédéterminé comprend au moins l'une d'entre : la comparaison de l'intervalle de pré-éjection ventriculaire gauche LPEI à un seuil maximal, et la comparaison du temps de remplissage diastolique FT à un seuil minimal ; et la comparaison du ratio entre intervalle de pré-éjection ventriculaire gauche LPEI et temps d'éjection ventriculaire gauche LVET à un seuil maximal, et la comparaison du temps de remplissage FT à un seuil minimal.

Le critère prédéterminé peut également comprendre la comparaison du ratio entre intervalle de pré-éjection ventriculaire gauche LPEI et temps d'éjection ventriculaire gauche LVET à un seuil maximal.

Le dispositif est de préférence apte à réajuster aussi bien le délai AV que le délai VV, à l'intérieur de valeurs limites respectives prédéterminées de délai AV et de délai VV, avantageusement à réajuster en priorité le délai AV si celui-ci n'a pas déjà atteint l'une des valeurs limites de délai AV, et à réajuster le délai VV si le délai AV a déjà atteint l'une de ces valeurs limites de délai AV. En réponse à la détection d'une amélioration de l'état hémodynamique du patient à partir des paramètres hémodynamiques, il est en outre possible d'allonger le délai AV et/ou raccourcir le délai VV.

On va maintenant décrire un exemple de mise en oeuvre du dispositif de l'invention, en référence aux dessins annexés.
La Figure 1 est un organigramme synoptique expliquant les principes de base de la mise en oeuvre de l'invention.
La Figure 2 est un organigramme détaillé explicitant la manière dont les paramètres DAV et DVV peuvent être modifiés dans le cadre de cette mise en oeuvre.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Ovatio* et *Paradym* produits et commercialisés par ELA Médical, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le cycle cardiaque est caractérisé par un certain nombre de paramètres hémodynamiques, parmi lesquels :
- l'intervalle de pré-éjection ventriculaire gauche ou LPEI (*Left Pre-Ejection Interval*),
- le temps d'éjection ventriculaire gauche ou LVET (*Left Ventricular Ejection Time*),
- le temps de remplissage diastolique FT ou DFT *(Diastolic Filling Time).* On connaît diverses techniques pour déterminer ces divers paramètres, à partir notamment du temps écoulé au cours d'un cycle entre la dépolarisation cardiaque (spontanée ou stimulée) et les différentes phases hémodynamiques constituées successivement par : le temps de pré-éjection, la contraction isovolumique, l'éjection systolique, la relaxation isovolumique et le remplissage de la cavité.

Ces instants du cycle peuvent être déterminés par divers capteurs tels que ceux évoqués dans le EP 0 862 927 A1 précité, ou encore par une technique décrite dans le EP 2 092 885 A1, où les différents marqueurs temporels des instants caractéristiques du cycle cardiaque sont déterminés par l'analyse d'un signal d'accélération endocardiaque, paramètre mesuré par un accéléromètre en contact avec le muscle cardiaque, notamment un capteur intégré à une sonde endocavitaire. Les données fournies par un tel capteur reflètent en effet très précisément et en temps réel les phénomènes concourant au fonctionnement mécanique du coeur et permettent ainsi, après filtrage et analyse, de fournir des marqueurs temporels de la systole et d'autres indices de performance hémodynamique du myocarde.

Ces paramètres peuvent être déterminés en temps réel, battement après battement, ce qui permet d'estimer à chaque instant les performances hémodynamiques du coeur et optimiser sans délai la thérapie appliquée au patient.

On considérera que l'optimisation de l'hémodynamique cardiaque doit répondre à au moins deux critères, par exemple (et de façon non limitative) les deux critères suivants :
- un temps de remplissage FT maximal, idéalement FT > 40 % (le temps de remplissage FT, qui est l'intervalle de temps séparant la fermeture de la valvule aortique de la fermeture de la valvule mitrale, est généralement exprimé de manière relative, en pourcentage de la durée complète d'un cycle (durée RR)) ;
- un délai systolique assurant un temps d'éjection maximal, donc un rapport LPEI/LVET minimal, idéalement LPEI/LVET < 34 %.

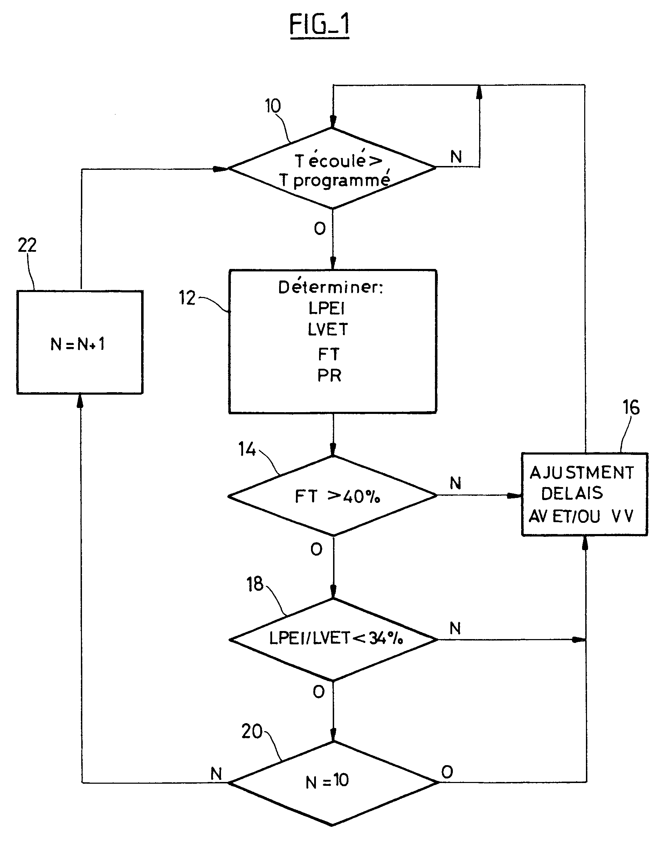

Sur la Figure 1, le bloc 10 indique un délai d'attente préprogrammé, correspondant à la périodicité à laquelle est effectuée la détermination des paramètres hémodynamiques et le réajustement des délais AV et/ou VV. A l'issue de ce délai, le dispositif détermine (bloc 12) les paramètres hémodynamiques nécessaires à l'optimisation, notamment LPEI, LVET, FT et PR.

En premier lieu, le temps de remplissage FT est examiné (test 14) pour voir s'il est supérieur à un seuil prédéterminé, par exemple le seuil de 40 % évoqué plus haut.

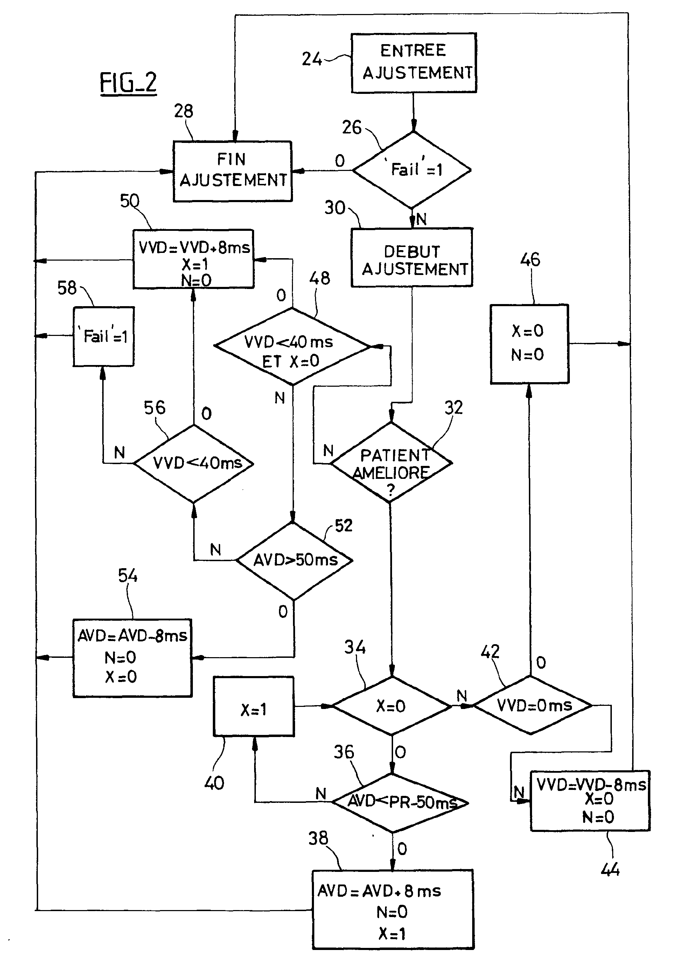

Dans la négative, ceci signifie que l'état hémodynamique du patient n'est pas satisfaisant, et le dispositif procède alors à un ajustement des délais AV et/ou VV (bloc 16, qui sera explicité en référence à la Figure 2).

Dans l'affirmative, ceci signifie que le temps de remplissage est correct, et le dispositif vérifie alors (test 18) si l'éjection est satisfaisante, par exemple en testant le ratio LPEI/LVET par rapport à un seuil prédéterminé tel que le seuil de 34 % évoqué plus haut.

Si ce dernier critère n'est pas vérifié, ceci signifie que l'éjection n'est pas satisfaisante et que la situation du patient peut être améliorée par ajustement des délais AV et/ou VV (bloc 16 précité).

Dans le cas contraire, le processus retourne au délai d'attente du bloc 10, après avoir incrémenté un compteur N (bloc 22) dont le rôle sera précisé plus loin.

Si le compteur N atteint une limite déterminée, par exemple N = 10 (test 20), alors les délais AV et/ou VV sont systématiquement réajustés en tant que de besoin (retour au bloc 16 précité).

Il est ainsi possible de modifier ces paramètres si l'on peut constater une amélioration lente de l'état du patient, sous l'effet d'un *"remodeling* cardiaque", dans le sens (i) de l'allongement du délai AV tout en assurant la capture complète du ventricule gauche, et (ii) de la réduction du délai VV au minimum, idéalement à zéro (situation où les deux ventricules se contractent de manière synchrone).

Sur la Figure 2, on a illustré les diverses étapes du processus d'ajustement des délais AV et VV, correspondant aux opérations exécutées au bloc 16 de la Figure 1.

Le processus commence au bloc 24,en testant un indicateur "Fail" qui est un marqueur d'échec positionné de la manière qui sera indiquée plus loin (bloc 58 de l'organigramme), pour indiquer que l'ajustement dynamique des paramètres n'a pas permis d'obtenir une amélioration de l'état du patient : il n'est alors pas nécessaire d'aller plus loin (test 26), et le processus prend fin dès ce stade (bloc 28).

En revanche, si le patient n'est pas en situation d'échec, le processus d'ajustement des délais AV et VV peut être engagé (bloc 30).

Si l'état du patient s'est amélioré au cours des dix itérations de l'organigramme de la Figure 1 (test 32), alors le processus examine un indicateur "X" (test 34) qui permet de décider de commencer l'ajustement en priorité par le délai AV, pour continuer ensuite sur le délai VV : si l'indicateur X est à zéro, cela signifie que l'ajustement doit commencer par le délai AV. Cet indicateur X permet ainsi, en cas d'amélioration des paramètres, de modifier alternativement le délai AV ou le délai VV.

Si ce délai AV n'est pas trop long, c'est-à-dire si par exemple il est inférieur au temps de conduction PR diminué de 50 ms (test 36), alors le délai AV est allongé d'un incrément, par exemple un incrément de 8 ms (bloc 38). En même temps, le compteur N (la valeur qui est examinée au test 20 de la Figure 1) est remis à zéro, et l'indicateur X est positionné à 1, pour indiquer qu'il y aura ensuite lieu d'ajuster le délai VV. Le processus d'ajustement est alors terminé (retour au bloc 28).

Si, à l'étape 36, le délai AV avait été testé comme trop long (AVD ≥ PR - 50 ms), alors l'indicateur X est positionné à 1 (bloc 40), sans modification du délai AV et sans remise à zéro du compteur N.

Lorsque, à l'étape 34, le processus trouve un indicateur X à 1, ceci signifie que le dispositif a déjà ajusté, ou tenté d'ajuster, le délai AV, et que c'est maintenant le délai VV qu'il convient d'ajuster.

Si ce délai VV est différent de sa valeur minimale VVD = 0 (test 42), alors le délai VV est réduit d'un incrément, par exemple un incrément de 8 ms (bloc 44). Simultanément, l'indicateur X est positionné à zéro et le compteur N remis à zéro. Le processus d'ajustement est alors terminé (retour au bloc 28).

Si le test 42 a indiqué un délai VV à zéro, ce délai VV n'est pas modifié, le dispositif repositionne à zéro l'indicateur X, remet à zéro le compteur N (bloc 46) et met fin au processus (retour au bloc 28).

Les ajustements des délais AV et VV que l'on vient de décrire sont opérés si, comme indiqué plus haut, le dispositif a déterminé à l'étape 32 une amélioration effective de l'état du patient.

Dans le cas contraire, le dispositif procède différemment, en exécutant les étapes 48 à 58.

A l'étape 48, le dispositif détermine s'il se trouve dans une situation où le délai VV n'est pas trop long (par exemple VVD < 40 ms) et si l'indicateur X est positionné à zéro (test 48).

Dans l'affirmative, le délai VV est augmenté d'un incrément, l'indicateur X positionné à 1 et le compteur N remis à zéro (bloc 50).

Dans la négative, le délai AV est examiné pour vérifier s'il est supérieur ou non à la valeur minimale susceptible d'être programmée, par exemple 50 ms (test 52).

Dans l'affirmative, le délai AV est réduit d'un incrément, l'indicateur X positionné à zéro et le compteur N remis à zéro (bloc 54).

Dans la négative, le dispositif détermine si le délai VV est inférieur à la valeur maximale susceptible d'être programmée (test 56).

Dans l'affirmative, ce délai VV est augmenté d'un incrément, l'indicateur X positionné à 1 et le compteur N remis à zéro (bloc 50 précité).

Dans la négative, le marqueur d'échec "Fail" est positionné à 1 (bloc 58), indiquant que l'état du patient s'est dégradé malgré l'allongement des délais AV et VV au maximum de ce qu'il est possible de programmer.

Dans ce dernier cas, seule une intervention du médecin pourra modifier la situation pour tenter d'améliorer l'état du patient.

## Revendications

1. Un dispositif médical actif implantable du type prothèse cardiaque de stimulation, resynchronisation et/ou défibrillation, comportant :
- des moyens de stimulation atrioventriculaire et/ou biventriculaire, aptes à appliquer au cours d'un même cycle cardiaque un délai atrioventriculaire entre un événement atrial et une stimulation ventriculaire, et/ou un délai interventriculaire entre deux stimulations ventriculaires ;
- des moyens capteurs aptes à délivrer au cours d'un même cycle cardiaque au moins deux paramètres hémodynamiques différents corrélés à des intervalles de temps représentatifs de la succession des phases systoliques et des phases diastoliques ;
- des moyens pour ajuster le délai atrioventriculaire et/ou le délai interventriculaire en fonction dudit paramètre hémodynamique ; et
- des moyens, opérant dynamiquement et de façon répétée sur plusieurs cycles cardiaques, pour :
déterminer (12) lesdits au moins deux paramètres hémodynamiques différents,
vérifier (14, 18) la concordance de ces paramètres hémodynamiques avec au moins un critère prédéterminé, et
dans la négative, réajuster en réponse (16) le délai atrioventriculaire et/ou le délai interventriculaire, dispositif **caractérisé en ce que** ledit au moins un critère prédéterminé comprend au moins l'un des premier et second critère, le premier critère étant défini par
- la comparaison de l'intervalle de pré-éjection ventriculaire gauche à un seuil maximal, et la comparaison du temps de remplissage diastolique à un seuil minimal, et le second critère étant défini par la comparaison du ratio entre intervalle de pré-éjection ventriculaire gauche et temps d'éjection ventriculaire gauche à un seuil maximal, et la comparaison du temps de remplissage diastolique à un seuil minimal.

2. Le dispositif de la revendication 1, dans lequel ledit au moins un critère prédéterminé comprend en outre la comparaison du ratio entre intervalle de pré-éjection ventriculaire gauche et temps d'éjection ventriculaire gauche à un seuil maximal.

3. Le dispositif de la revendication 1, dans lequel le dispositif comprend des moyens pour réajuster aussi bien le délai atrioventriculaire que le délai interventriculaire, à l'intérieur de valeurs limites respectives prédéterminées de délai atrioventriculaire et de délai interventriculaire.

4. Le dispositif de la revendication 3, dans lequel le dispositif comprend des moyens pour réajuster en priorité le délai atrioventriculaire si celui-ci n'a pas déjà atteint l'une des valeurs limites de délai atrioventriculaire, et pour réajuster le délai interventriculaire si le délai atrioventriculaire a déjà atteint l'une de ces valeurs limites de délai atrioventriculaire.

5. Le dispositif de la revendication 1, comprenant des moyens pour détecter une amélioration de l'état hémodynamique du patient à partir desdits paramètres hémodynamiques, et en réponse allonger le délai atrioventriculaire et/ou raccourcir le délai interventriculaire.

## Claims

1. Implantable active medical device of the cardiac stimulation, resynchronization and/or defibrillation prosthesis type comprising:
- atrioventricular and/or biventricular stimulation means, capable of applying, during one and the same cardiac cycle, an atrioventricular delay between an atrial event and a ventricular stimulation, and/or an interventricular delay between two ventricular stimulations:
- sensing means capable of delivering, during one and the same cardiac cycle, at least two different haemodynamic parameters correlated at time intervals representative of the succession of the systolic phases and of the diastolic phases;
- means for adjusting the atrioventricular delay and/or the interventricular delay as a function of the said haemodynamic parameter; and
- means, operating dynamically and in a repeated manner over several cardiac cycles, for:
• determining (12) the said at least two different haemodynamic parameters,
• verifying (14, 18) that these haemodynamic parameters concord with at least one predetermined criterion, and
• if they do not, readjusting in response (16) the atrioventricular delay and/or the interventricular delay, which device is **characterized in that** the said at least one predetermined criterion comprises at least one of the first and second criteria, the first criterion being defined by:
• the comparison of the interval of left ventricular pre-ejection with a maximum threshold, and the comparison of the diastolic filling time with a minimum threshold, and the second criterion being defined by the comparison of the ratio between left ventricular pre-ejection interval and left ventricular ejection time with a maximum threshold, and the comparison of the diastolic filling time with a minimum threshold.

2. Device of Claim 1, in which the said at least one predetermined criterion also comprises the comparison of the ratio between left ventricular pre-ejection interval and left ventricular ejection time with a maximum threshold.

3. Device of Claim 1, in which the device comprises means for readjusting both the atrioventricular delay and the interventricular delay inside respective predetermined limit values of atrioventricular delay and interventricular delay.

4. Device of Claim 3, in which the device comprises means for readjusting, in priority, the atrioventricular delay if the latter has not already reached one of the limit values of atrioventricular delay and in order to readjust the interventricular delay if the atrioventricular delay has already reached one of these limit values of atrioventricular delay.

5. Device of Claim 1, comprising means for detecting an improvement in the haemodynamic state of the patient based on the said haemodynamic parameters and in response for lengthening the atrioventricular delay and/or shortening the interventricular delay.

## Patentansprüche

1. Implantierbare aktive medizinische Vorrichtung vom Typ Herzprothese zur Stimulation, Resynchronisation und/oder Defibrillation, die aufweist:
- Einrichtungen zur atrioventrikulären und/oder biventrikulären Stimulation, die während eines gleichen Herzzyklus eine atrioventrikuläre Verzögerung zwischen einem atrialen Ereignis und einer ventrikulären Stimulation und/oder eine interventrikuläre Verzögerung zwischen zwei ventrikulären Stimulationen anwenden können,
- Sensoreinrichtungen, die während eines gleichen Herzzyklus mindestens zwei unterschiedliche hämodynamische Parameter liefern können, die mit Zeitintervallen korreliert sind, die für die Aufeinanderfolge der systolischen Phasen und der diastolischen Phasen repräsentativ sind;
- Einrichtungen, um die atrioventrikuläre Verzögerung und/oder die interventrikuläre Verzögerung in Abhängigkeit vom hämodynamischen Parameter einzustellen; und
- Einrichtungen, die dynamisch und wiederholt über mehrere Herzzyklen arbeiten, um:
• die mindestens zwei verschiedenen hämodynamischen Parameter zu bestimmen (12),
• die Konkordanz dieser hämodynamischen Parameter mit mindestens einem vorbestimmten Kriterium zu prüfen (14, 18), und
• bei negativem Ergebnis als Reaktion (16) die atrioventrikuläre Verzögerung und/oder die interventrikuläre Verzögerung nachzustellen, Vorrichtung, die **dadurch gekennzeichnet ist, dass** das mindestens eine vorbestimmte Kriterium mindestens eines der ersten und zweiten Kriterien enthält, wobei
• das erste Kriterium durch den Vergleich des linksventrikulären Präejektionsintervalls mit einem maximalen Schwellwert und den Vergleich der diastolischen Füllzeit mit einem minimalen Schwellwert definiert ist,
• und das zweite Kriterium durch den Vergleich des Verhältnisses zwischen linksventrikulärem Präejektionsintervall und linksventrikulärer Ejektionszeit mit einem maximalen Schwellwert und den Vergleich der diastolischen Füllzeit mit einem minimalen Schwellwert definiert ist.

2. Vorrichtung nach Anspruch 1, bei der das mindestens eine vorbestimmte Kriterium außerdem den Vergleich des Verhältnisses zwischen linksventrikulärem Präejektionsintervall und linksventrikulärer Ejektionszeit mit einem maximalen Schwellwert enthält.

3. Vorrichtung nach Anspruch 1, bei der die Vorrichtung Einrichtungen enthält, um sowohl die atrioventrikuläre Verzögerung als auch die interventrikuläre Verzögerung innerhalb von vorbestimmten Grenzwerten der atrioventrikulären Verzögerung bzw. der interventrikulären Verzögerung nachzustellen.

4. Vorrichtung nach Anspruch 3, bei der die Vorrichtung Einrichtungen enthält, um prioritär die atrioventrikuläre Verzögerung nachzustellen, wenn diese nicht bereits einen der Grenzwerte der atrioventrikulären Verzögerung erreicht hat, und um die interventrikuläre Verzögerung nachzustellen, wenn die atrioventrikuläre Verzögerung bereits einen der Grenzwerte der atrioventrikulären Verzögerung erreicht hat.

5. Vorrichtung nach Anspruch 1, die Einrichtungen enthält, um eine Verbesserung des hämodynamischen Zustands des Patienten ausgehend von den hämodynamischen Parametern zu erfassen und als Reaktion die atrioventrikuläre Verzögerung zu verlängern und/oder die interventrikuläre Verzögerung zu verkürzen.
